(19) Europäisches Patentamt
European Patent Office
Office européen des brevets

(11) Veröffentlichungsnummer: **0 149 173**
**B1**

(12) # EUROPÄISCHE PATENTSCHRIFT

(45) Veröffentlichungstag der Patentschrift: **08.04.87**

(51) Int. Cl.⁴: **C 07 C 143/11**, C 07 C 139/00, E 21 B 43/22

(21) Anmeldenummer: **84115672.2**

(22) Anmeldetag: **18.12.84**

(54) **Tributylphenoletherglycidylsulfonate, Verfahren zu deren Herstellung und deren Verwendung.**

(30) Priorität: **30.12.83 DE 3347578**

(43) Veröffentlichungstag der Anmeldung: **24.07.85 Patentblatt 85/30**

(45) Bekanntmachung des Hinweises auf die Patenterteilung: **08.04.87 Patentblatt 87/15**

(84) Benannte Vertragsstaaten: **BE DE FR GB NL**

(56) Entgegenhaltungen:
**EP-A-0 003 183**
**EP-A-0 116 929**
**DE-A-2 748 721**
**DE-C-2 724 490**
**GB-A-1 566 655**
**US-A-3 977 471**
**US-A-4 077 471**
**US-A-4 088 189**
**US-A-4 222 957**
**US-A-4 318 816**

(73) Patentinhaber: **HOECHST AKTIENGESELLSCHAFT, Postfach 80 03 20, D-6230 Frankfurt am Main 80 (DE)**

(72) Erfinder: **Uhl, Klaus, Dr., Am Dachsbau 1, D-6232 Bad Soden am Taunus (DE)**
Erfinder: **Gulden, Walter, Dr., Frankfurter Strasse 70, D-6238 Hofheim am Taunus (DE)**
Erfinder: **Wester, Norbert, Dr., Sachsenring 12, D-6238 Hofheim am Taunus (DE)**
Erfinder: **Schneider, Gerhart, Dr., Ziegelheck 3, D-6240 Königstein/Taunus (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Bei der Gewinnung von Öl aus unterirdischen Lagerstätten gelingt es im allgemeinen nur 20 - 30 % des ursprünglich vorhandenen Öls durch primäre Gewinnungsverfahren zu fördern. Hierbei gelangt das Öl mit Hilfe des natürlichen Lagerstättendrucks an die Erdoberfläche. Bei der sekundären Gewinnung wird Wasser in die geologische Formation eingepreßt und das Öl durch mehrere Produktionssonden gefördert. Dieses Wasserfluten als Sekundärmaßnahme ist relativ billig und wird daher häufig verwendet, führt jedoch in vielen Fällen nur zu einer geringen Mehrentölung der Lagerstätte.

Nach Beendigung der sekundären Erdölfördermaßnahmen ist allein durch Zufuhr mechanischer Energie keine weitere wirtschaftliche Erdölgewinnung zu erreichen. In dem heterogenen Porenraum überholt das niedriger viskose Wasser das höher viskose Öl, so daß fast nur noch Wasser und kein Öl mehr gefördert wird. Hat der Verwässerungsgrad bei ca. 98 % die Wirtshnaftlichkeitsgrenze überschritten, so kommen nur noch Verfahren der tertiären Gewinnung in Frage. Darunter versteht man Verfahren, bei denen entweder die Viskosität des Öls erniedrigt und/oder die Viskosität des nachflutenden Wassers erhöht und/oder die Grenzflächenspannung zwischen Wasser und Öl erniedrigt werden.

Die meisten Verfahren lassen sich als thermische Ölgewinnungsverfahren, Lösungs- oder Mischfluten, Tensid- oder Polymerfluten bzw. als Kombination von mehreren der genannten Verfahren einordnen. Bei thermischen Gewinnungsverfahren wird Dampf oder heißes Wasser in die Lagerstätte injiziert oder es erfolgt eine in situ-Verbrennung. Beim Lösungs- oder Mischungsverfahren wird Lösungsmittel für das Erdöl (Gas oder eine Flüssigkeit) in die Lagerstätte injiziert. Tensidflutverfahren beruhen auf einer starken Erniedrigung der Grenzflächenspannung zwischen Öl und Flutwasser. Je nach Tensidkonzentration unterscheidet man Tensidfluten (Low tension flooding), micellares Fluten und Emulsionsfluten.

In der Monografie von D.O. Shah und R.S. Schechter: "Improved Oil Recovery by Surfactant and Polymerflooding, Academic Press Inc." sowie in zahlreichen Patentschriften werden eine Vielzahl von Tensiden aufgeführt, die beim Tensidflutprozeß Verwendung finden können. Als Tenside werden dabei vor allem Sulfonate wie z.B. synthetische und natürliche Petrolsulfonate MG 350 - 500, Alkylsulfunate wie z.B. $C_{13-20}$ sec Alkansulfonat-Na MG 328/350, α-Olefinsulfonat-Na ($C_{15}$-$C_{30}$), Alkylarylsulfonate, wie z.B. Dodecylbenzolsulfonat-Na, Alkyltoluolsulfonate oder Alkylxylolsulfonate beschrieben. Diese Sulfonate besitzen eine sehr niedrige Toleranzgrenze gegenüber dem Salzgehalt der Lagerstättengewässer.

So sind z.B. Petrolsulfonate nur in einem Wasser mit einem Salzgehalt von 1,5 % NaCl löslich.

Sulfonate sind vor allem auch gegen die im Lagerstättenwasser enthaltenen Erdalkaliionen sehr empfindlich. Bei höheren Salzkonzentrationen bilden sich beim Einsatz dieser Tenside Fällungsprodukte, die zur Verstopfung des porösen Raumes der Formation führen können. Viele Lagerstättenwässer besitzen höhere Salinitäten z.B. in Norddeutschland bis zu 25 %. Um in diesen Lagerstättensystemen mit Sulfonaten arbeiten zu können, wurden Kombinationen mit Alkoholen und/oder nichtionischen Tensiden (Alkylpolyglykolether bzw. Alkylarylpolyglykolether) vorgeschlagen, die bei diesen Salzkonzentrationen stabil sind, aber meist wurde die ölmobilisierende Wirkung verschlechtert.

Gegenstand der Erfindung sind neue Tributylphenoletherglycidylsulfonate der Formel

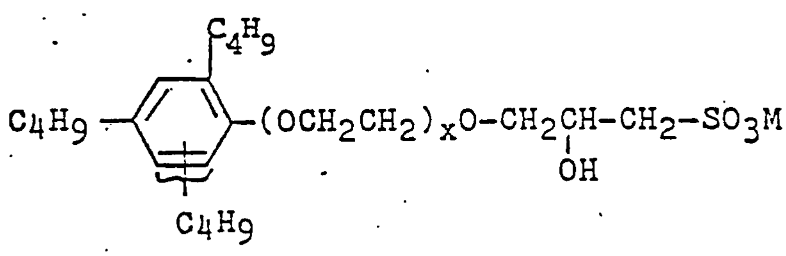

worin x eine Zahl von 2 bis 20, vorzugsweise 2 bis 12 und M $K^+$, $Na^+$, $NH4^+$ oder das Kation einer Aminbase bedeuten sowie ein Verfahren zu deren Herstellung, das darin besteht, daß man eine Verbindung der Formel

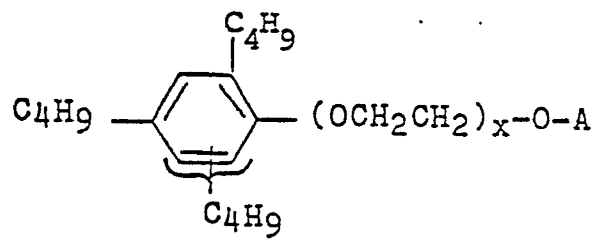

worin A eine Gruppe der Formel

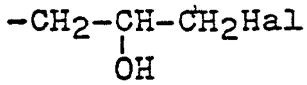 oder 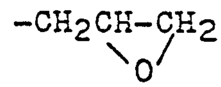

und Hal Chlor oder Brom bedeuten, mit Kalium- oder Natriumsulfit bzw. Natriumhydrogensulfit oder Kaliumhydrogensulfit umsetzt und gegebenenfalls das erhaltene Kalium- oder Natriumsalz in ein anderes Salz unter der Bedeutung von M umwandelt.

In der US-PS 3 827 497 wird die Verwendung der Salze sulfatierter oxyalkylierter Alkohole vorgeschlagen. Es hat sich aber gezeigt, daß Salze, die die C-O-S-Bindung enthalten, bei erhöhten Temperaturen hydrolytisch gespalten werden. In den US-Patenten 4 018 278, 4 088 189, 4 077 471, 4 110 228, 4 318 816, 4 194 564, 4 318 816, 3 977 471 werden Ethersulfonate ($C_{12}$-$C_{15}$ Alkyl- und Alkylarylethersulfonate) beschrieben, die im Lagerstättenwasser mit hohem Salzgehalt (200 g/l) und bei einer Lagerstättentemperatur bis zu 120°C beständig sind. Mit Hilfe dieser Tenside kann aber nur bei ausgewählten Lagerstätten eine ölmobilisierende Wirksamkeit festgestellt werden. Ähnliche Erfahrungen wurden mit den in der DOS 2 724 442 und DOS 2 724 490 beschriebenen Produkten Alkyletherpropansulfonat- bzw. Alkyletherglycerylsulfonat gemacht.

Die einzelnen Lagerstätten unterscheiden sich in der Temperatur, die zwischen 20 und 120°C liegen kann, in der Ölzusammensetzung, das paraffinisch, naphthenisch, aromatisch, hoch-, mittel- oder niederviskos sein kann, im Salzgehalt und der Salzzusammensetzung der zwischen 3 % und 25,0 % NaCl mit unterschiedlichem Gehalt an Erdalkalien liegen kann, in der Lagerstättenformation, der minerologischen Zusammensetzung (z.B. Sandstein oder Kalkstein), Porosität und Permeabilität. Ziel der Entwicklungsarbeit ist es daher, Tenside zu finden, die bei möglichst allen vorhandenen Lagerstättenbedingungen (d.h. im Temperaturbereich von 20 bis 120° C, Salzgehalt 30 bis 250 g/l) und bei den verschiedenen Öltypen wirksam sind.

Überraschenderweise wurde gefunden, daß Tri-butylphenoletherglycidylsulfonate bei der Verwendung als Tenside für die Erdölförderung den oben beschriebenen Verbindungen überlegen und bei unterschiedlichen Lagerstättenparametern wirksam sind.

Die Verbindungen der obigen Former leiten sich her vom Tributylphenol, vorzugsweise von technisch verfügbaren Mischungen aus 2,4,6-Tributylphenol und einem geringeren Anteil von 2,4,5-Tributylphenol, wobei diese Mischung weiterhin noch geringe Mengen Dibutylphenol und Tetrabutylphenol enthält. Ein Beispiel hierfür ist eine Mischung bestehend aus 1,4 - 2 % 2,6-dibutyl-phenol, 0,3 - 0,8 % 2,4-dibutylphenol, 1,2 - 2,2 % 2,5-dibutylphenol, 70 - 80 % 2,4,6-Tributylphenol, 9 - 12 % 2,4,5-tributylphenol und 6 - 7 % 2,4,5,6-tetrabutylphenol wobei der Gesamtanteil der drei dibutyl-Isomeren ca. 3 % beträgt.

Die Ethersulfonate, besonders auch die Tributylphenolethersulfonate der obigen Formel sind Tenside, die sich durch Beständigkeit in einem weiten Temperatur, und pH-Bereich auszeichnen. Wäßrige Lösungen dieser Verbindungen erniedrigen die Oberflächenspannung an der Phasengrenze Tensidlösung/Luft auf Werte in der Größenordnung von 25 bis 30 $mNm^{-1}$ und die Grenzflächenspannung Öl/Tensidlösung auf Werte von 1 bis 10 $mMm^{-1}$ (nach Du Nouy). Aufgrund ihrer Wirksamkeit und Stabilität über einen weiten pH-Bereich eignen sich diese Verbindungen für alkalische und für saure Metallreinigungsverfahren.

Sie sind außerdem wirksame Emulgiermittel für die Emulsions-Polymerisation und eignen sich als Stabilisatoren für Latex und andere Polymer-Emulsionen. Von besonderem Interesse ist auch die Verwendung dieser Verbindungen bei der tertiären Erdölförderung und bei der Sondenstimulierung und Frac-behandlung von Erdöl-Lagerstätten. Der Einsatz der Verbindung gemäß der vorliegenden Erfindung erhöht bei diesen Verfahren die Ölausbeute. In allen Fällen wird das Tensid im allgemeinen in Mengen von 0,01 bis 10, vorzugsweise 0,05 bis 3 % eingesetzt.

Die Herstellung der erfindungsgemäßen Verbindungen erfolgt üblicherweise in zwei Stufen entsprechend

dem folgenden Reaktionsschema:

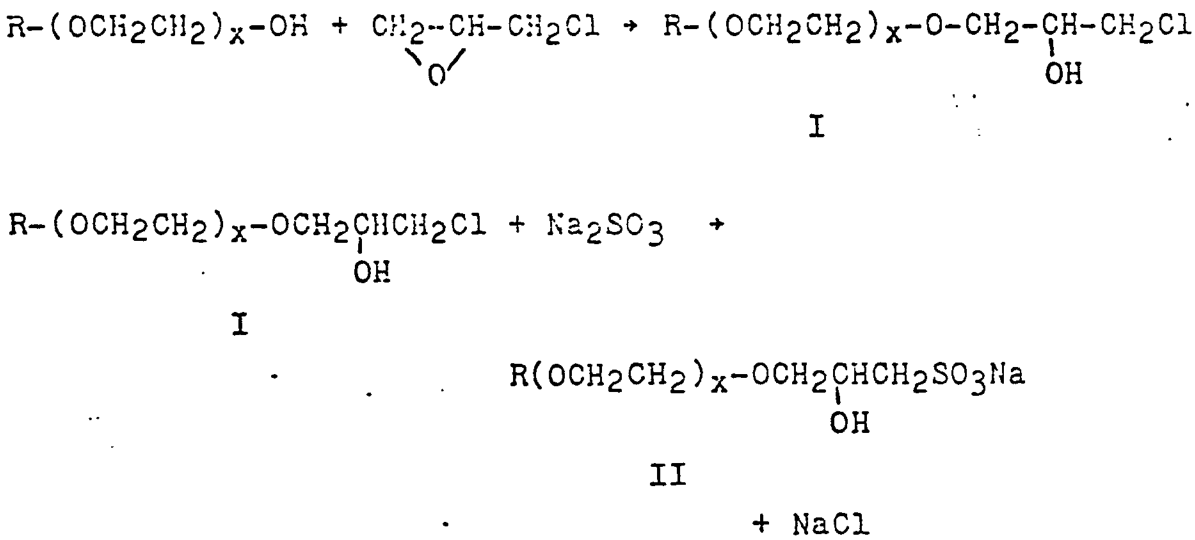

I

I

II

Im ersten Reaktionsschritt wird unter Verwendung saurer Katalysatoren wie $BF_3$, $SnCl_4$ oder $H_2SO_4$ Epichlorhydrin an den entsprechenden Tributylphenolpolyglykolether addiert. Die Reaktion wird vorzugsweise ohne Lösungsmittel und bei 70 - 80°C durchgeführt. Es kann jedoch auch in einem inerten Lösungsmittel wie Toluol gearbeitet werden, ohne Ausbeuteverluste hinnehmen zu müssen. Die so gewonnene Zwischenverbindung I wird dann bei 120 - 200°C vorzugsweise bei 160° - 180°C mit $Na_2SO_3$ in wäßriger Lösung gegebenenfalls unter Zuhilfenahme eines geeigneten Solubilisierungsmittels (vergl. Deutsche Patentanmeldung P 33 05 328.6) wie Diethylenglykol zum Sulfonat II umgesetzt.

Eine weitere Synthesemöglichkeit besteht darin, daß man die Zwischenverbindung I entsprechend der Gleichung

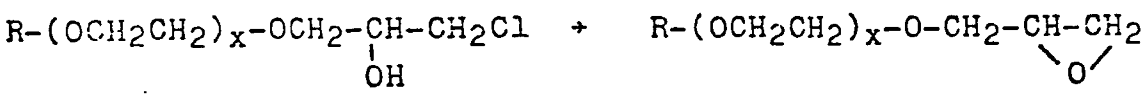

I III

mit einer starken Base die KOH oder NaOH zum entsprechenden Glycidether III umsetzt. Hieraus erhält man in einem 2. Schritt durch Addition von $NaHSO_3$ in wäßriger Lösung die Glycidylethersulfonate in guten Ausbeuten.

**Beispiele**

1a) 1 Chlor-2-hydroxipropyl-(tri-butylphenolhexaox-ethyl)ether der Formel

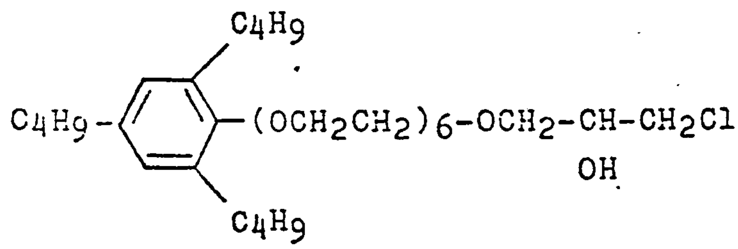

400 g (0.75 Mol) Tri(butylphenolhexa-oxethylether werden unter Stickstoffatmosphäre auf 75° C erhitzt. Nach Zugabe von katalytischen Mengen $BF_3$-Etherat tropft man ohne weitere Heizung 72,0 g (0,78 Mol) Epichlorhydrin bei dieser Temperatur zu und rührt zur Vervollständigung der Reaktion noch 2 Stunden bei 70 - 80° C nach. Man überführt das gesamte Reaktionsgemisch in einen beheizbaren Scheidetrichter und wäscht heiß mit gesättigter $NaHCO_3$-, dann mit gesättigter NaCl-Lösung. Man erhält ein hellbraunes Öl, das zur Entfernung von Restwasser im Vakuum bei 80° C über $P_2O_5$ getrocknet wird.

Das Produkt wird durch folgende Analysendaten charakterisiert:

OH-Zahl 80 (Theorie 86)

Gesamt Cl 5,0 % (Theorie 5,5 %)

Nach dem in Beispiel 1a) beschriebenen Verfahren werden hergestellt:

2a) 1 Chlor-2-hydroxipropyl-(tri-butylphenoltetra-oxethyl)-ether

Das Produkt wird durch folgende Kennzahlen charakterisiert:

OH-Zahl 94 (Theorie 103)

Gesamtchlor 6,0 % (Theorie 6,5 %)

3a) 1 Chlor-2-hydroxiproyyl-(tri-butylphenoloctaox-ethyl)-ether

Das Produkt wird durch folgende Kennzahlen charakterisiert:

OH-Zahl 71 (Theorie 79)

Gesamtchlor 4,6 % (Theorie 5 %)

1b) Herstellung von Tributylphenolhexaoxethylglycidyl-ethersulfonat-Na

286 g (0,44 Mol) 1-Chlor-2-hydroxipropyl-(tri-butyl-phenolhexa-oxethyl)-ether,

64 g (0,48 Mol) $Na_2SO_3$ 95 %, 52,5 g Diethylenglykol, 1 Spatelspitze NaJ und 473 g E-Wasser werden bei einem Anfangs-pH-Wert von 10 - 11 während 5 Stunden bei 175°C gerührt. Man erhält eine hochviskose Flüssigkeit, die, bestimmt durch die Zweiphasentitration (Mischindikator, Dimidiumbromid/Disulfinblau 2:1, 0,005 normale Hyamin-1622 Lösung), einen Wirksubstanzgehalt von 24,1 % aufweist.

Nach dem in Beispiel 1b) beschriebenen Verfahren wurden die folgenden Verbindungen hergestellt aus dem Vorprodukten der Beispiele 2a und 3a:

2b) Tri-butylphenoltetraoxethylglycidylethersulfonat-Na. Wirksubstanzgehalt 26,2 %

3b) Tri-butylphenoloctaoxethylglycidylethersulfonat-Na. Wirksubstanzgehalt 23,7 %

Zur Bestimmung der Wirksamkeit der erfindungsgemäßen Verbindungen wird die in der US-Patentschrift 4 008 165 beschriebene Mikrokapillarentölungs-Methode und die Bestimmung der Grenzflächenspannung nach der Spinningdrop-interfacial-Tensiometer-Methode herangezogen.

Bei der Mikrokapillarentölung werden als Modell für den Porenraum der Lagerstätte Mikrokapillaren aus Glas der Firma Drummond Scientific Co./USA verwendet, die bei einem Volumen von 5 µl eine Länge von 30 mm und einen Durchmesser von 0,45 mm aufwiesen.

Die Mikrokapillaren werden an einem Ende abgeschmolzen, in einem Exsikator evakuiert und mit Rohöl gefüllt. Die Kapillaren werden in Tensidlösungen (Reagenzgläser), die im Wasserbad temperiert werden, mit der Öffnung nach oben senkrecht eingebracht und die Verdrängung des Öls wird in Abhängigkeit von der Zeit visuell registriert.

Mit Hilfe des folgenden Beurteilungsschemas kann die Wirksamkeit der Tenside in Abhängigkeit von dessen Konzentration, der Salzkonzentration, pH-Wert, Temperatur und Ölzusammensetzung bestimmt werden.

Wert
9 leer (30 mm) nach 10 Minuten
8 leer nach 1 Stunde
7 leer nach 3 Stunden
6 leer nach 20 Stunden
5 16 - 25 mm Entleerung nach 20 Stunden
4 9 - 15 mm Entleerung nach 20 Stunden
3 4 - 8 mm Entleerung nach 20 Stunden
2 1 - 3 mm Entleerung nach 20 Stunden
1 Spur Entleerung nach 20 Stunden
0 unverändert nach 20 Stunden

Diese Methode bietet den Vorteil, daß bei dem geringen Durchmesser der Mikrokapillaren, Viskosität und Dichte der Öle keinen großen Einfluß auf die Entölungswirkung ausüben und es möglich ist, mit Lagerstättenöl und Lagerstättenwasser zu arbeiten.

Nach Taber J. Petr. Techn. 3 (1969), S. 3 - 12 sind Tenside für die tertiäre Erdölgewinnung nur geeignet, wenn die Grenzflächenspannung an der Phasengrenze Öl/Salzwasser auf Werte kleiner $10^{-2}$ $mNm^{-1}$ erniedrigt wird. Für diese Bestimmung der Grenzflächenspannung an der Phasengrenze Öl/Wasser wird das vom Wade und Burkowsky entwickelte Spinning-Drop-Interfacial-Tensiometer verwendet. (M. Burkowsky und C. Marx: Über den Mechanismus des Tensidflutens in hochsalinaren Systemen; Erdöl-Erdgas-Zeitschrift 95 (1979), S. 17 - 25.

Die Methode beruht darauf, daß ein Öltropfen, der in eine um die horizontale Achse rotierende Kapillare gebracht wird, die eine Flüssigkeit (Salzwasser + Tensid) mit höherer Dichte enthält, deformiert wird. Der Tropfen wird gestreckt, bis ein Gleichgewicht der deformierenden Kräfte und der Grenzflächenspannung erreicht wird.

Die Grenzflächenspannung errechnet sich nach Vonnegut (B. Vonnegut, Rev. Sci Instruments 13 (1942), S. 6-9) aus dem gemessenen Öltropfendurchmesser R, der Rotationsgeschwindigkeit W und dem Dichteunterschied $\Delta d$ nach folgender Formel:

$$\gamma\, 1/2 = \frac{\Delta d \cdot W^2 \cdot R^3}{4}$$

Die mit diesen Methoden gemessenen Werte sind in den folgenden Tabellen zusammengefaßt. Es wurden in allen Fällen jeweils 1%ige wäßrige Lösungen der Tenside verwendet. Als Vergleichsprodukte gemäß dem Stand der Technik wurden die Ethersulfonate von Nonylphenol und Cocos-Fettalkohol mit getestet.

**I. Test mit Lagerstättenöl K**

Salzgehalt 180 g/l NaCl, 20 g/l $CaCl_2$, pH 8,5 Temperatur 40° C

| | Grenzflächenspannung [$mNm^{-1}$] | Mikrokapillarentölung |
|---|---|---|
| 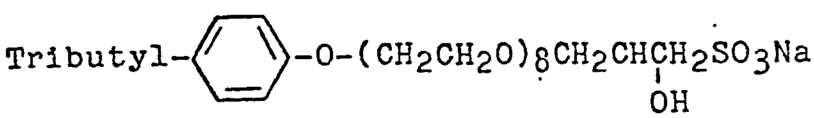 | $6 \cdot 10^{-4}$ | 9 |
| Cocos-Alkyl-O-$(CH_2CH_2O)_3CH_2CH(OH)CH_2SO_3Na$ | $1 \cdot 10^{-1}$ | 0 |

**II. Test mit Lagerstättenöl K**

Salzgehalt 100 g/l NaCl, pH 8,5, Temperatur 40° C

| | Grenzflächenspannung [$mNm^{-1}$] | Mikrokapillarentölung |
|---|---|---|
| 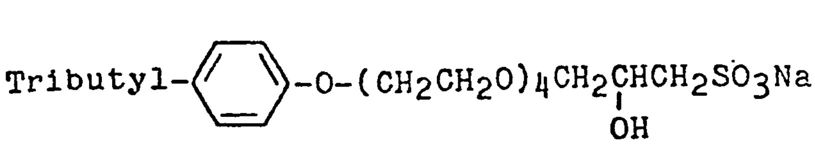 | $1 \cdot 10^{-3}$ | 8 |

**III. Test mit einem Paraffinischen Öl**

Salzgehelt 135 g/l NaCl, 15 g/l $CaCl_2$, pH 8,5 Temperatur 60° C

| | Grenzflächen-spannung [$mNm^{-1}$] | Mikrokapil-larentölung |
|---|---|---|
| 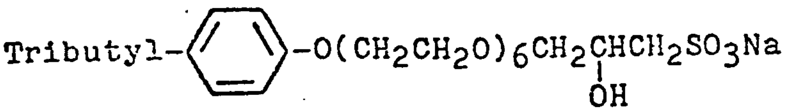 | $5 \cdot 10^{-4}$ | 9 |
| Cocos-Alkyl-O-$(CH_2-CH_2O)_3CH_2\underset{OH}{\underset{\mid}{C}}HCH_2SO_3Na$ | $5 \cdot 10^{-2}$ | 0 |
| 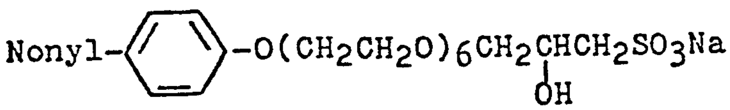 | $1 \cdot 10^{-1}$ | 0 |

**IV Test mit einem aromatischen Öl**
Salzgehalt 70 g/l NaCl, 30 g/l $CaCl_2$, pH 6,5 Temperatur 80° C

| | Grenzflächen-spannung [$mNm^{-1}$] | Mikrokapil-larentölung |
|---|---|---|
| 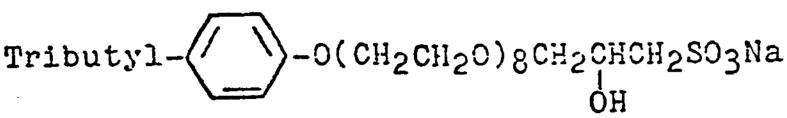 | $7 \cdot 10^{-3}$ | 8 |
| Cocos-Alkyl-O-$(CH_2CH_2O)_3CH_2\underset{OH}{\underset{\mid}{C}}HCH_2SO_3Na$ | $4{,}1 \cdot 10^{-2}$ | 0 |
| 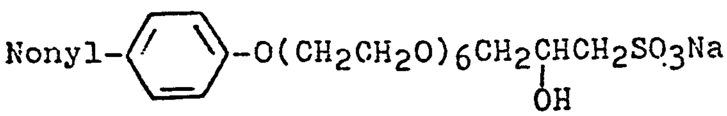 | $4{,}2 \cdot 10^{-2}$ | 0 |

**V. Test mit einem aromatischen Öl**
Salzgehalt 135 g/l NaCl 15 g/l $CaCl_2$, pH 8,5 Temperatur 40° C

| | Grenzflächen-spannung [mNm$^{-1}$] | Mikrokapil-larentölung |
|---|---|---|
| 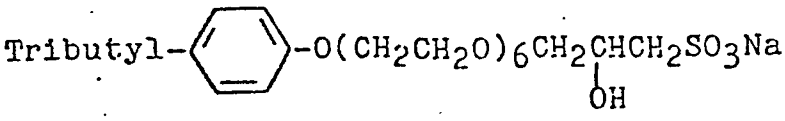 | $6 \cdot 10^{-5}$ | 9 |
| 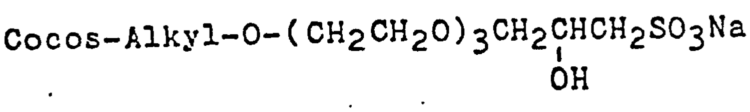 | $2 \cdot 10^{-2}$ | 0 |
| 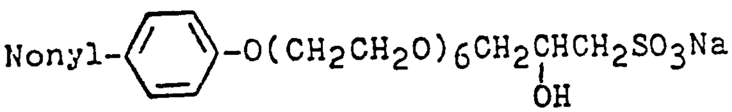 | $1 \cdot 10^{-1}$ | 0 |

**VI. Test mit einem aromatischen Öl**
Salzgehalt 105 g/l NaCl, 45 g $CaCl_2$, pH 8,5 Temperatur 40° C

| | Grenzflächen-spannung [mNm$^{-1}$] | Mikrokapil-larentölung |
|---|---|---|
| 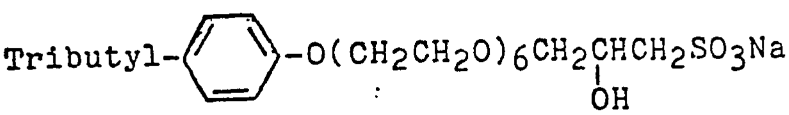 | $1 \cdot 10^{-5}$ | 9 |
| 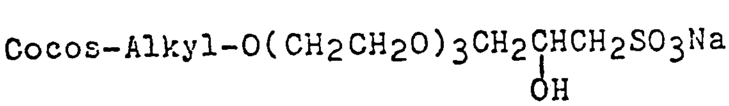 | $2 \cdot 10^{-2}$ | 0 |
| 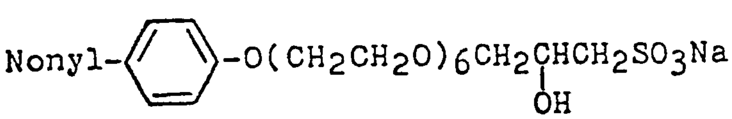 | $1 \cdot 10^{-1}$ | 0 |

**VII. Test mit einem naphthenischen Öl**
Salzgehalt 135 g/1 NaCl 15 g/l $CaCl_2$, pH 8,5 Temperatur 60° C

| | Grenzflächenspannung [$mNm^{-1}$] | Mikrokapillarentölung |
|---|---|---|
| Tributyl-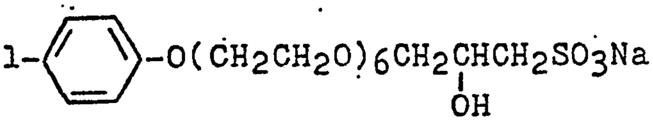 | $4 \cdot 10^{-4}$ | 9 |
| Cocos-Alkyl-O-$(CH_2CH_2O)_3CH_2\underset{OH}{CH}CH_2SO_3Na$ | $6 \cdot 10^{-2}$ | 0 |
| Nonyl-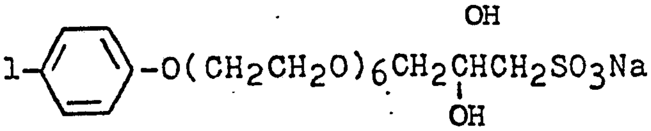 | $1 \cdot 10^{-1}$ | 0 |

**VIII Test mit einem naphthenischen Öl**

Salzgehalt 140 g/l $NaCl_2$, 60 g/1 $CaCl_2$, pH 6,5 Temperatur 60° C

| | Grenzflächenspannung [$mNm^{-1}$] | Mikrokapillarentölung |
|---|---|---|
| Tributyl-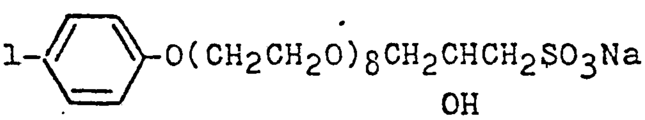 | $5 \cdot 10^{-1}$ | 9 |
| Cocos-Alkyl-O$(CH_2CH_2O)_3CH_2\underset{OH}{CH}CH_2SO_3Na$ | $1 \cdot 10^{-2}$ | 0 |
| Nonyl-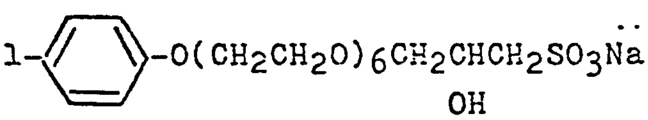 | $4 \cdot 10^{-2}$ | 0 |

**IX. Test mit einem paraffinischen (1) naphthenischen (2) und aromatischen Öl (3)**

Salzgehalt jeweils 150 g/l NaCl, pH 6,5, Temperatur 80° C

| | Grenzflächenspannung $[mNm^{-1}]$ (1) | (2) | (3) |
|---|---|---|---|
| 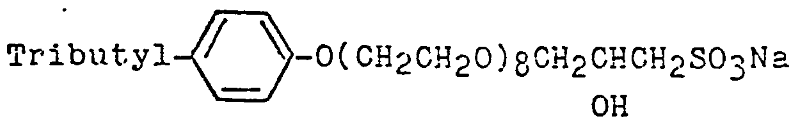 | $4 \cdot 10^{-3}$ | $5 \cdot 10^{-3}$ | $8,5 \cdot 10^{-3}$ |
| Cocos-Alkyl-$O(CH_2CH_2O)_3CH_2CH(OH)CH_2SO_3Na$ | $1,8 \cdot 10^{-2}$ | $1,2 \cdot 10^{-2}$ | $1,2 \cdot 10^{-2}$ |
| 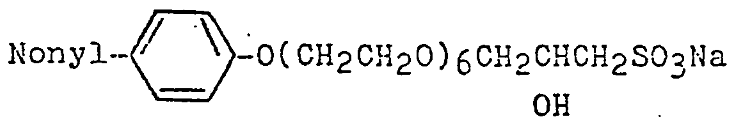 | $1,2 \cdot 10^{-2}$ | $7 \cdot 10^{-2}$ | $6,8 \cdot 10^{-2}$ |

Die Tabellen zeigen, daß die Tributylphenoletherglycidylsulfonate bei unterschiedlichen Salzgehalten (50 - 200 g/l) den bereits bekannten Verbindungen in der Öl mobilisierung überlegen sind.

Die Ethersulfonate können auch in Kombination mit anderen anionischen Tensiden, wie z.B. Petrolsulfonaten, sec. Alkansulfonaten, α-Olefinsulfonaten, Dodecylbenzolsulfonaten und nichtionischen Tensiden wie Alkyl- bzw. Alkylphenolpolyglykolethern eingesetzt werden. Als weitere Zusätze kommen Alkohole und Glykolether in Frage. Die Viskosität des Flutwassers kann außerdem durch Polymere, wie z.B. Hydroxiethylcellulose, Polyacrylamide oder Polysaccaride erhöht werden.

## Patentansprüche

1. Tributylphenoletherglycidylsulfonate der Formel

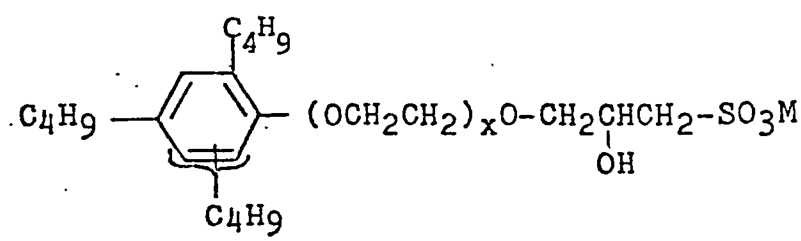

worin x eine Zahl von 2 bis 20 und M $Na^+$, $K^+$, $NH_4^+$ oder das Kation einer Aminbase bedeuten.

2. Verfahren zur Herstellung der Tributylphenoletherglycidylsulfonate nach Anspruch 1, dadurch gekennzeichnet, daß man eine Verbindung der Formel

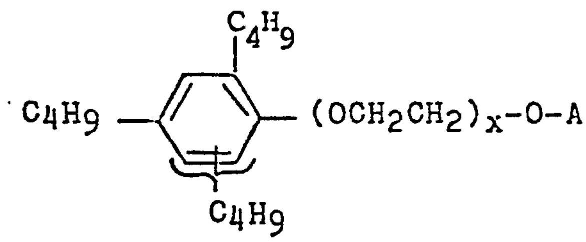

worin A eine Gruppe der Formel

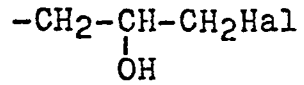 oder 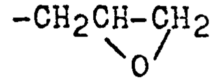

und Hal Chlor oder Brom bedeuten, mit Kalium- oder Natriumsulfit bzw. Natriumhydrogensulfit oder Kaliumhydrogensulfit umsetzt und gegebenenfalls das erhaltene Kalium- oder Natriumsalz in ein anderes Salz unter der Bedeutung von M umwandelt.

3. Verwendung der Tributylphenoletherglycidylsulfonate nach Anspruch 1 für die Erdölförderung.

**Claims**

1. Tributylphenoletherglycidylsulfonates of the formula

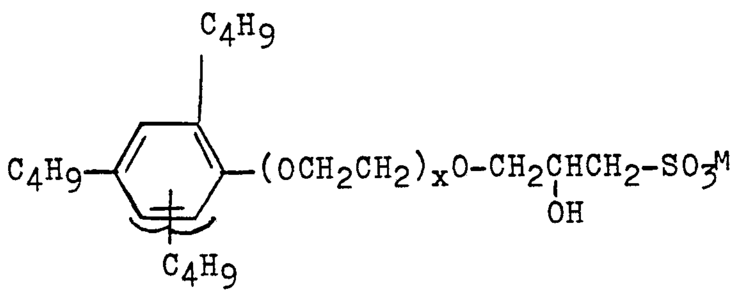

wherein x is an integer from 2 to 20 and M is $Na^+$, $K^+$, $NH_4^+$ or the cation of an amine.

2. Process for preparing tributylphenoletherglycidylsulfonates of claim 1, which process is characterized by reacting a compound of the formula

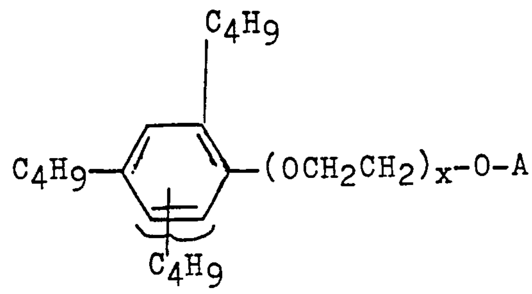

wherein A ist a group of the formula

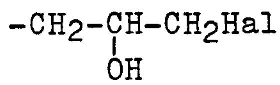

or

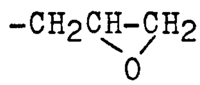

and Hal is chlorine or bromine, with potassium- or sodium sulfite, sodium hydrogensulfite or potassium hydrogensulfite and optionally converting the potassium- or sodium salt thus obtained into a different salt under the definition of M.

3. Use of tributylphenoletherglycidylsulfonates according to claim 1 in the crude oil recovery.

**Revendications**

1. Ethers glycidyliques sulfonés de produits de polyéthoxylation de tributyl-phénols qui répondent à la formule suivante:

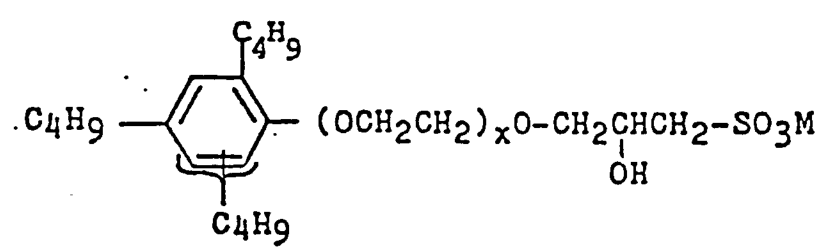

dans laquelle x représente un nombre de 2 à 20, et M représente Na+ K+ $NH_4$+ ou le cation d'une amine.

2. Procédé de préparation d'éthers glycidyliques sulfonés de produits de polyéthoxylation de tributylphénols selon la revendication 1, procédé caractérisé en ce qu'on fait réagir un composé répondant à la formule:

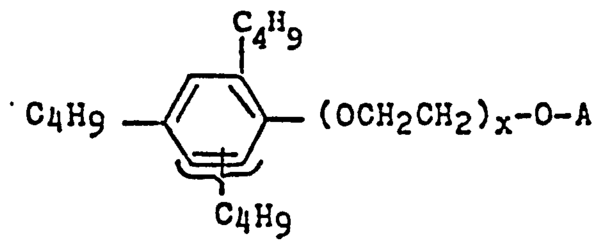

dans laquelle A représente un radical répondant à l'une des formules:

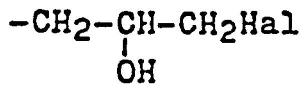

ou

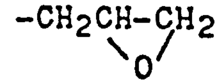

(où Hal représente le chlore ou le brome), avec le sulfite de potassium ou de sodium ou l'hydrogénosulfite de sodium ou de potassium, et on transforme éventuellement le sel de potassium ou de sodium ainsi obtenu en un autre sel correspondant à la signification de M.

3. Applications des éthers glycidyliques sulfonés de produits de polyéthoxylation de tributyl-phénols selon la revendication 1 pour l'extraction du pétrole.